# EUROPÄISCHE PATENTSCHRIFT

(11) **EP 0 988 384 B1**
(45) Veröffentlichungstag und Bekanntmachung des Hinweises auf die Patenterteilung: **14.09.2005**
(21) Anmeldenummer: 98942588.9
(22) Anmeldetag: 21.07.1998
(51) Int. Cl.: C12N 15/56, C12N 9/42, C12N 9/24, C12P 21/02, C12N 1/21

(54) **NEUE BETA-GLUCANASE AUS BACILLUS**
NEW BETA-GLUCANASE FROM A BACILLUS
NOUVELLE BETA-GLUCANASE OBTENUE A PARTIR D'UN BACILLE

(30) Priorität: 30.07.1997 DE 19732751
(43) Veröffentlichungstag der Anmeldung: 29.03.2000
(73) Patentinhaber: Henkel Kommanditgesellschaft auf Aktien, 40589 Düsseldorf (DE)
(72) Erfinder: HILLEN, Wolfgang, D-91080 Uttenreuth (DE); MAURER, Karl-Heinz, D-40699 Erkrath (DE)
(86) Internationale Anmeldenummer: PCT/EP1998/004564
(87) Internationale Veröffentlichungsnummer: WO 1999/006573

(56) Entgegenhaltungen:
- TABENERO CARLOS PILAR: "Cloning and DNA sequencing of bgaA, a gene encoding an endo-beta-1,3-1,4-*glucanase*, from an alkalophilic *Bacillus* strain (N137)." APPLIED AND ENVIRONMENTAL MICROBIOLOGY, 1994, XP002089122
- LOUW ME ET AL: "Characterization, cloning and sequencing of a thermostable endo-(1,3-1,4) *beta*-*glucanase*-encoding gene from an *alkalophilic* *Bacillus* brevis." APPL MICROBIOL BIOTECHNOL, JAN 1993, 38 (4) P507-13, XP002089123 GERMANY
- HORIKOSHI K ET AL: "Production of 1, 3-*beta*-*glucanase* by *Bacillus* No. 221. An *alkalophilic* microorganism." BIOCHIM BIOPHYS ACTA, APR 19 1975, 384 (2) P477-83, XP002089124 NETHERLANDS

## Beschreibung

Die vorliegende Erfindung betrifft ein Enzym, das Mischglucane, die alternierend in 1,3-und 1,4-β-glucosidischer Bindung verknüpft sind, hydrolytisch in Oligosaccharide spalten kann sowie den Mikroorganismus, der dieses Enzym bildet.

Derartige Enzyme gehören zur Klasse der Endo-1,3-1,4-β-D-glucan-4-glucanohydrolasen (EC 3.2.1.73; Lichenasen) oder der Endo-1,3-β-D-glucosidasen (EC 3.2.1.39; Laminarinasen). Im Sinne der vorliegenden Erfindung wird ein solches Enzym hier als β-Glucanase oder Beta-Glucanase bezeichnet.

Polymere Mischglucane der oben genannten Art sind in unterschiedlichen Anteilen in praktisch allen Getreideprodukten enthalten. Enzyme, welche diese zu spalten vermögen, werden vor allem in der Nahrungsmittel-, Getränke- und Futtermittelindustrie, der Textilindustrie und der Stärkeverarbeitung benötigt (R. Borriss, "β-Glucan-spaltende Enzyme", in H. Ruttloff, "Industrielle Enzyme", Kapitel 11.5, Behr's Verlag, Hamburg, 1994). Eine der wichtigsten Anwendungen von β-Glucanasen liegt im Bereich der Getränke- und Brauindustrie, wo derartige Enzyme zum Abbau von Malz- und Gersten-β-Glucan eingesetzt werden. Die hierfür zum Einsatz kommenden Enzyme stammen üblicherweise aus *Bacillus subtilis*, wie zum Beispiel in der deutschen Patentschrift DD 226 012 A1 beschrieben, oder *Bacillus amyloliquefaciens*, doch sind auch β-Glucanasen aus anderen Mikroorganismen, beispielsweise *Achromobacter lunatus, Arthrobacter luteus, Aspergillus aculeatus, Aspergillus niger, Disporotrichum dimorphosporum, Humicola insolens, Penicillium emersonii, Penicillium funiculosum* oder *Trichoderma reesei,* bekannt. Ein handelsübliches Produkt wird zum Beispiel unter der Bezeichnung Cereflo® (Hersteller: Novo Nordisk A/S) zum Einsatz in der Brauereiindustrie angeboten.

Bisher bekannte β-Glucanasen weisen pH-Optima im schwach sauren bis neutralen Bereich auf, so daß ihre Verwendung auf Verfahren beschränkt ist, die bei diesen pH-Werten durchgeführt werden.

Eine Endo-β-1,3-1,4-Glucanase mit einem pH-Optimum von 7,5 und Stabilität im alkalischen Bereich geht aus der Publikation "Cloning and DNA Sequencing of *bga*A, a Gene Encoding an Endo-β-1,3-1,4-Glucanase, from an Alkalophilic *Bacillus* Strain (N137)" von C. Tabernero et al. (1994) in *Appl. Envir. Microbiol.,* Band 60, Seiten 1213-1220 hervor. Sie weist zu der in der vorliegenden Anmeldung beschriebenen β-Glucanase auf DNA-Ebene eine Identität von 67,4% und auf Protein-Ebene eine Identität v on 65,0% Identität auf, jeweils für das komplette Präproprotein.

Die Anmelderin hatte sich zum Ziel gesetzt, das
Anwendungsgebiet der β-Glucanasen zu erweitern und eine β-Glucanase zu entwickeln, die im Hinblick auf eine Verwendung in technischen Prozessen, die im alkalischen Milieu stattfinden, eine ausreichende Stabilität auch unter diesen Bedingungen aufweist.

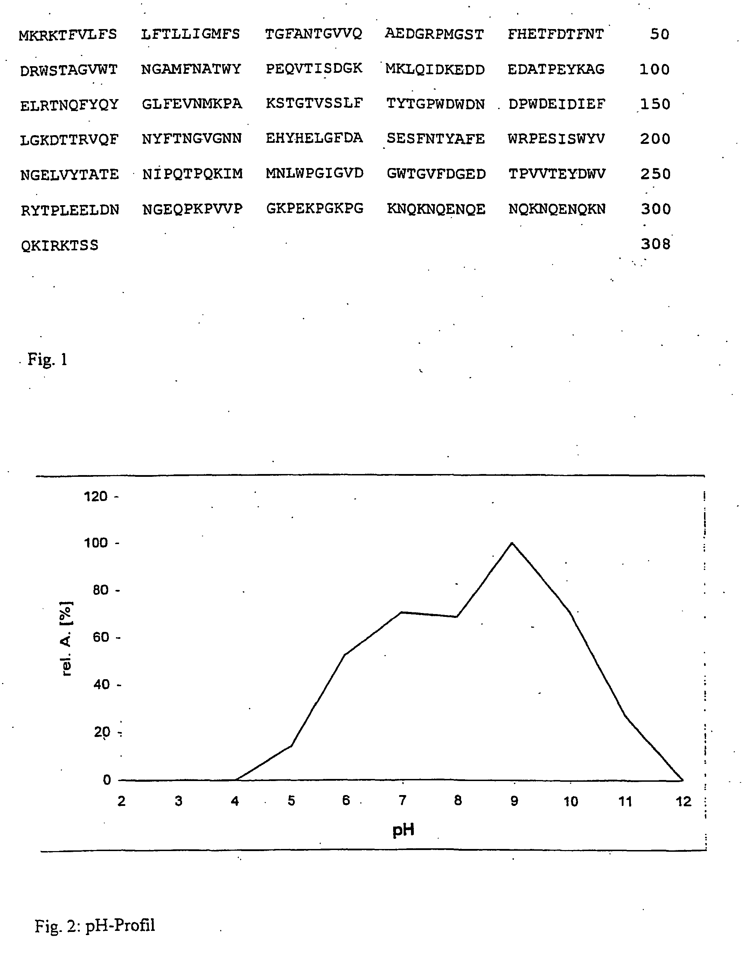

Gegenstand der vorliegenden Erfindung ist ein Enzym welches β-glucanolytische Aktivität aufweist, dadurch gekennzeichnet, daß es die in Fig. 1 wiedergegebene Aminosäuresequenz aufweist, der eine β-Glucanase erzeugende Mikroorganismus *Bacillus alkatophilus* DSM 9956 und das für die genannte β-Glucanase kodierende Gen, das im Rahmen der Arbeiten, die zur vorliegenden Erfindung führten, identifiziert und sequenziert (SEQ ID-NO.:2) worden ist. Dieses Gen kann gewünschtenfalls in im Prinzip bekannter Weise in anderen Bakterien kloniert und dort die β-Glucanase exprimiert werden. Ein weiterer Gegenstand der Erfindung sind daher auch durch mikrobiologische Verfahren erhältliche Mikroorganismen, welche das genannte Gen enthalten. Die aus der Sequenz des β-Glucanase-Gens aus *Bacillus alkalophilus* DSM 9956 abgeleitete Aminosäuresequenz der aus diesem Mikroorganismus erhältlichen erfindungsgemäßen β-Glucanase (SEQ ID-NO:1) ist in Fig. 1 im Ein- Buchstaben-Code wiedergegeben. Die β-Glucanase aus *Bacillus alkalophilus* DSM 9956 einschließlich des Signalpeptids, das nach dem Transport durch die Zellwand des Mikroorganismus durch eine Signalpeptidase abgespalten wird und das nach Vergleich mit literaturbekannten Daten [M.E. Louw, S.J. Reid, Watson, Appl. Microbiol. Biotech. 39 (1993), 507-513] vermutlich 31 Aminosäuren umfasst, besteht aus 308 Aminosäuren. Der entsprechende Mikroorganismus ist Grampositiv, seine Zellform ist stäbchenförmig (Breite ca. 0,7 µm bis 0,9 µm, Länge ca. 2,5 µm bis 4,0 µm); er ist von der Patentanmelderin am 13.4.1995 bei der DSM-Deutsche Sammlung von Mikroorganismen und Zellkulturen GmbH, Mascheroder Weg 1b, 38124 Braunschweig hinterlegt worden und hat die Hinter- legungsnummer DSM 9956 erhalten.

Vorzugsweise weist eine erfindungsgemäße β-Glucanase eine Homologie von über 70%, insbesondere 75% bis 99% zur β-Glucanase aus *Bacillus alkatophilus* DSM 9956 auf. Entsprechendes gilt für das zugrundeliegende Gen.

Ein bevorzugtes Einsatzgebiet für das erfindungsgemäße Enzym liegt in der Lebensmittelindustrie, insbesondere der Getränke- und Brauereiindustrie und ist insbesondere die Verwendung zur Entfernung von Glucan und/oder Lichenan bei der Reinigung von Membranen und sonstigen Vorrichtungen dieser Industriezweige.

Ein weiterer Gegenstand der Erfindung ist ein Verfahren zur Entfernung von Glucan und/oder Lichenan bei der Reinigung von Membranen und sonstigen Vorrichtungen der Lebensmittelindustrie, insbesondere der Brauereündustrie unter Einsatz einer erfindungsgemäßen β-Glucanase.

### Beispiele

### Beispiel 1

Chromosomale DNA aus *Bacillus alkalophilus* C/M2-3 wurde mit Sau3A partiell verdaut und eine Fraktion von 4 bis 8 kb großen Fragmenten gelelektrophoretisch isoliert. Nach Ligation in die *Bam*H1-site des Plasmids pMK4, eines *E. coli - Bacillus* shuttle-Vektors [M.A. Sullivan et al., Gene 29 (1984), 21-26], wurde in kompetente *E. coli* DH5α - Zellen transforiniert. Rekombinante Klone mit β-Glucanaseaktivität wurden durch Congo-RotFärbung auf LB-Platten mit 0,2 % Lichenin (pH8.5) identifiziert.

Die β-Glucanase wurde aus dem Zellüberstand eines Klons in *E. coli* DH 5 α pmK4 aufgereinigt. Nach Dialyse des zellfreien Überstands gegen 20 mM Natriumphosphatpuffer (pH 7,5) wurde das Dialysat auf Q-Sepharose (Pharmacia) gebunden und mit einem linearen Gradienten von 0 - 1 M NaC1 in 25 mM Natriumphosphatpuffer (pH 7,5 oder pH 9,0) eluiert.

Der Nachweis und die Bestimmung der glucanolytischen Aktivität beruht auf Modifizierungen des von M. Lever in Anal. Biochem. 47 (1972), 273-279 und Anal. Biochem. 81 (1977), 21-27 beschriebenen Verfahrens. Dazu wird eine 0,5 gewichtsprozentige Lösung von β-Glucan (Sigma no. G6513) in 50 mM Glycinpuffer (pH 9,0) eingesetzt. 250 gl dieser Lösung werden zu 250 µl einer Lösung, die das auf glucanolytische Aktivität zu testende Mittel enthält, gegeben und 30 Minuten bei 40°C inkubiert. Anschließend werden 1,5 ml einer 1 gewichtsprozentigen Lösung von p- Hydroxybenzoesäurehydrazid (PAHBAH) in 0,5 M NaOH, die 1 mM Bismutnitrat und 1 mM Kaliumnatriumtartrat enthält, zugegeben und die Lösung wird 10 Minuten auf 70 °C erwärmt. Nach Abkühlen (2 Minuten 0 °C) wird bei Raumtemperatur die Absorption bei 410 nm (zum Beispiel unter Verwendung eines Photometers Uvikon® 930) unter Verwendung einer Glukose-Eichkurve gegenüber einem Blindwert bestimmt. Als Blindwert wird eine Lösung herangezogen, die wie die Meßlösung vorbereitet wurde mit dem Unterschied, daß man die Glucan-Lösung erst nach der Zugabe der PAHBAH-Lösung zugibt. 1 U entspricht der Enzymmenge, die unter diesen Bedingungen 1 µmol Glucose pro Minute erzeugt.

Die spezifische Aktivität des so erhaltenen Enzyms lag bei 4390 mU pro mg Protein, wohingegen die Ausgangslösung eine um den Faktor 152 geringere Aktivität aufgewiesen hatte. Das Enzym wurde als homogene Bande in der SDS-Polyacrylamid-Gelelektrophorese angefärbt (Silberfärbung).

Mit Hilfe von Markerproteinen (Cytochrom c, Pferde-Myoglobin, Chymotrypsinogen, Ovalbumin, Rinderserumalbumin) als internem Standard wurde mittels SDS-Polyacrylamid-Gelelektrophorese das Molekulargewicht der β-Glucanase aus *Bacillus alkalophilus* DSM 9956 zu ca. 30 000 abgeschätzt.

In der isoelektronischen Fokussierung (pH 3 bis 9) wurde der isoelektrische Punkt der β-Glucanase mittels Aktivitätsanfärbung zu pH 5,2 bestimmt.

### Beispiel 2: pH-Profil

Die Bestimmung der glucanolytischen Aktivität bei verschiedenen pH-Werten erfolgte in einem Davies-Universalpuffer (21,01 g Zitronensäure H₂O, 13,61 g KH₂PO₄, 19,07 g Na₂B₄O₇ · 10 H₂O, 12,11 g Tris und 7,46 g KCI in 1 l dest. Wasser; 50 ml dieser Stammlösung werden mit 0,4 N NaOH auf den gewünschten pH eingestellt und mit dest. Wasser auf 200 ml aufgefüllt) bei 40 °C und 30 minütiger Inkubation. Wie aus dem in Fig. 2 wiedergegebenen pH-Profil (Auftragung der relativen glukanolytischen Aktivität, rel. A., gegen den pH-Wert) deutlich wird, ist das Enzym am aktivsten im Bereich zwischen pH 6 und pH 10,5. Das Optimum liegt bei pH 9.

### Beispiel 3: Temperatur-Profil

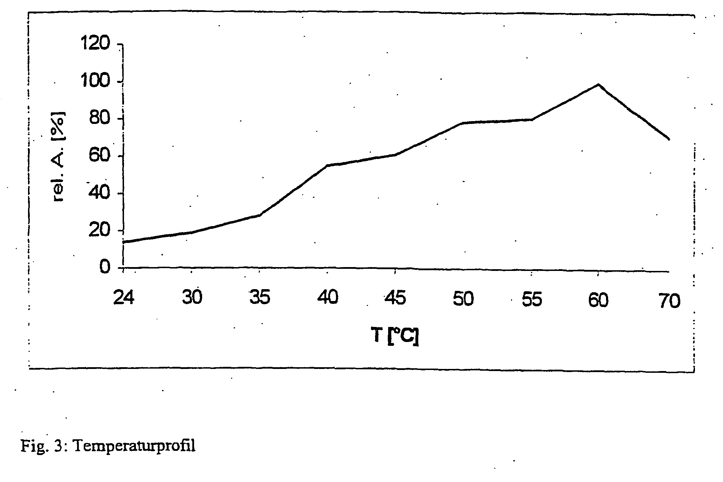

Die Temperaturabhängigkeit der glucanolytischen Aktivität der aus *Bacillus alkalophilus* DSM 9956 gewonnenen β-Glucanase wurde in Glycin/NaOH bei pH 9 mit 15 minütiger Inkubation gemessen. Der pH-Wert der Testlösung wurde angepasst, da der Puffer eine Temperaturabhängigkeit von ca. pH 0,033 pro °C aufweist. Das Maximum der glucanolytischen Aktivität liegt bei 60 °C, wie man aus Fig. 3 erkennt, in der die relative glukanolytische Aktivität (rel. A.) des Enzyms in Abhängigkeit von der Temperatur (T) aufgetragen ist.

### SEQUENZPROTOKOLL

**ALLGEMEINE ANGABEN:**
ANMELDER:

| | |
|---|---|
| NAME | Cognis Gesellschaft für Biotechnologie mbH |
| STRASSE | Henkelstraße 67, Gebäude Y 20 |
| OR | Düsseldorf |
| BUNDESLAND | Nordrhein-Westfalen |
| POSTLEITZAHL | 40589 |
| TELEFON | 0211 - 7976763 |
| TELEFAX | 0211 - 7987607 |

BEZEICHNUNG DER ERFINDUNG: Neue Beta-Glucanase aus Bacillus
ANZAHL DER SEQUENZEN: 2
BRIEF ADRESSE:

| | |
|---|---|
| ADRESSAT | Cognis Gesellschaft für Biotechnologie mbH |
| STRASSE | Henkelstraße 67, Gebäude Y 20 |
| ORT | Düsseldorf |
| BUNDESLAND | Nordrhein-Westfalen |
| POSTLEITZAHL | 40589 |

COMPUTERLESBARE FASSUNG:

| | |
|---|---|
| DATENTRÄGER | Diskette |
| COMPUTER | IBM PC-kompatibel |
| BETRIEBSSYSTEM | MS-WINDOWS |
| SOFTWARE | MS WORD 97 |

**ANGABEN ZU SEQ ID-NO:1:**
SEQUENZ-CHARACTERISTIKA:
   LÄNGE: 308 Aminosäuren
   ART: Aminosäure
   TOPOLOGIE: unbekannt
ART DES MOLEKÜLS: Protein
HYPOTHETISCH: NEIN
SEQUENZBESCHREIBUNG: SEQ ID-NO:1:

Met Lys Arg Lys Thr Phe Val Leu Phe Ser Leu Phe Thr Leu Leu Ile
1               5                   10                  15
Gly Met Phe Ser Thr Gly Phe Ala Asn Thr Gly Val Val Gln Ala Glu
            20                  25                  30
Asp Gly Arg Pro Met Gly Ser Thr Phe His Glu Thr Phe Asp Thr Phe
        35                  40                  45
Asn Thr Asp Arg Trp Ser Thr Ala Gly Val Trp Thr Asn Gly Ala Met
    50                  55                  60
Phe Asn Ala Thr Trp Tyr Pro Glu Gln Val Thr Ile Ser Asp Gly Lys
65                  70                  75                  80
Met Lys Leu Gln Ile Asp Lys Glu Asp Asp Glu Asp Ala Thr Pro Glu
                85                  90                  95
Tyr Lys Ala Gly Glu Leu Arg Thr Asn Gln Phe Tyr Gln Tyr Gly Leu
            100                 105                 110
Phe Glu Val Asn Met Lys Pro Ala Lys Ser Thr Gly Thr Val Ser Ser
        115                 120                 125
Leu Phe Thr Tyr Thr Gly Pro Trp Asp Trp Asp Asn Asp Pro Trp Asp
    130                 135                 140
Glu Ile Asp Ile Glu Phe Leu Gly Lys Asp Thr Thr Arg Val Gln Phe
145                 150                 155                 160
Asn Tyr Phe Thr Asn Gly Val Gly Asn Asn Glu His Tyr His Glu Leu
                165                 170                 175
Gly Phe Asp Ala Ser Glu Ser Phe Asn Thr Tyr Ala Phe Glu Trp Arg
            180                 185                 190
Pro Glu Ser Ile Ser Trp Tyr Val Asn Gly Glu Leu Val Tyr Thr Ala
        195                 200                 205
Thr Glu Asn Ile Pro Gln Thr Pro Gln Lys Ile Met Met Asn Leu Trp
    210                 215                 220
Pro Gly Ile Gly Val Asp Gly Trp Thr Gly Val Phe Asp Gly Glu Asp
225                 230                 235                 240
Thr Pro Val Val Thr Glu Tyr Asp Trp Val Arg Tyr Thr Pro Leu Glu
                245                 250                 255
Glu Leu Asp Asn Asn Gly Glu Gln Pro Lys Pro Val Val Pro Gly Lys
            260                 265                 270
Pro Glu Lys Pro Gly Lys Pro Gly Lys Asn Gln Lys Asn Gln Glu Asn
        275                 280                 285
Gln Glu Asn Gln Lys Asn Gln Glu Asn Gln Lys Asn Gln Lys Ile Arg
    290                 295                 300
Lys Thr Ser Ser
305         308

**ANGABEN ZU SEQ ID-NO:2:**
SEQUENZ-CHARACTERISTIKA:
   LÄNGE: 927 Basenpaare
   ART: Nukleinsäure
   STRANGFORM: Doppelt
   TOPOLOGIE: linear
ART DES MOLEKÜLS: Genom-DNA
HYPOTHETISCH: NEIN
ANTI-SENSE: NEIN
URSPRÜNGLICHE HERKUNFT:
   STAMM: Bacillus alkalophilus DSM 9956
SEQUENZBESCHREIBUNG: SEQ ID-NO:2:

ATG AAA AGG AAG ACA TTT GTA TTA TTT TCT TTA TTT ACG TTG TTA 45
ATT GGT ATG TTC TCA ACA GGG TTT GCA AAT ACA GGT GTG GTT CAG 90
GCA GAA GAT GGG AGA CCA ATG GGG TCG ACG TTT CAT GAA ACG TTT 135
GAT ACC TTT AAT ACG GAC CGC TGG TCA ACA GCT GGG GTA TGG ACA 180
AAT GGA GCA ATG TTT AAT GCG ACA TGG TAT CCA GAA CAG GTG ACC 225
ATT TCA GAT GGG AAA ATG AAG TTG CAA ATT GAC AAG GAA GAT GAT 270
GAA GAT GCA ACC CCA GAA TAT AAG GCT GGG GAA TTA AGA ACG AAT 315
CAG TTT TAT CAA TAC GGG TTG TTT GAA GTC AAT ATG AAG CCA GCG 360
AAA TCA ACA GGA ACC GTC TCT TCA CTC TTT ACA TAT ACG GGT CCA 405
TGG GAT TGG GAT AAT GAT CCT TGG GAT GAA ATC GAT ATT GAG TTC 450
CTT GGA AAG GAT ACA ACA AGA GTC CAA TTT AAC TAT TTT ACT AAC 495
GGA GTA GGA AAC AAT GAA CAT TAC CAC GAA TTA GGG TTC GAT GCA 540
TCA GAA TCT TTT AAT ACG TAT GCT TTT GAA TGG AGA CCA GAA TCA 585
ATT AGT TGG TAC GTA AAC GGA GAA TTA GTA TAT ACA GCA ACA GAA 630
AAT ATC CCG CAA ACA CCA CAA AAA ATT ATG ATG AAC TTA TGG CCT 675
GGA ATT GGA GTG GAT GGA TGG ACA GGC GTT TTT GAC GGA GAA GAC 720
ACT CCA GTT GTA ACG GAG TAT GAT TGG GTA AGG TAC ACT CCA CTA 765
GAG GAA TTA GAT AAT AAC GGA GAA CAA CCG AAA CCT GTA GTG CCA 810
GGA AAA CCA GAA AAA CCA GGA AAA CCA GGG AAA AAC CAG AAA AAC 855
CAG GAA AAC CAG GAA AAC CAG AAA AAC CAG GAA AAC CAG AAA AAC 900
CAA AAA ATC AGA AAA ACC AGT AGT TGA 927

## Patentansprüche

1. β-Glucanase erzeugender Mikroorganismus Bacillus alkalophilus DSM 9956.

2. Enzym, welches β-glucanolytische Aktivität aufweist, **dadurch gekennzeichnet, daß** es die in Fig. 1 wiedergegebene Aminosäuresequenz aufweist.

3. Enzym welches β-glucanolytische Aktivität aufweist, **dadurch gekennzeichnet, daß** es eine Homologie von über 70 % zu der des Enzyms mit der in SEQ ID-NO:1 wiedergegebenen Aminosäuresequenz aufweist.

4. Enzym nach Anspruch 3, **dadurch gekennzeichnet, daß** es eine Homologie von 75 % bis 99 % zu der des Enzyms mit der in SEQ ID-NO:1 wiedergegebenen Aminosäuresequenz aufweist.

5. Gen, welches für ein Enzym mit β-glucanolytischer Aktivität kodiert, **dadurch gekennzeichnet, daß** es die in SEQ ID-NO. :2 wiedergegebene Sequenz oder eine Homologie von über 70 % zu dieser Sequenz aufweist.

6. Gen nach Anspruch 5, **dadurch gekennzeichnet, daß** es eine Homologie von 75 % bis 99 % zu der in SEQ ID-NO.:2 wiedergegebenen Sequenz aufweist.

7. Durch mikrobiologische Verfahren erhältlicher Mikroorganismus, welcher ein Gen gemäß einem der Ansprüche 5 bis 6 enthält.

8. Verwendung eines β-glucanolytische Aktivität aufweisenden Enzyms, das die in Fig. 1 wiedergegebene Aminosäuresequenz aufweist, zur Entfernung von Glucan und/oder Lichenan bei der Reinigung von Membranen und Vorrichtungen der Lebensmittelindustrie, insbesondere der Brauereiindustrie.

9. Verwendung eines β-glucanolytische Aktivität aufweisenden Enzyms, das eine Homologie von über 70 % zu der des Enzyms mit der in SEQ ID-NO: 1 wiedergegebenen Aminosäuresequenz aufweist, zur Entfernung von Glucan und/oder Lichenan bei der Reinigung von Membranen und Vorrichtungen der Lebensmittelindustrie, insbesondere der Brauereiindustrie.

10. Verwendung nach Anspruch 9, **dadurch gekennzeichnet, daß** das β-glucanolytische Aktivität aufweisende Enzym eine Homologie von 75 % bis 99 % zu der des Enzyms mit der in SEQ ID-NO: 1 wiedergegebenen Aminosäuresequenz aufweist.

11. Verfahren zur Entfernung von Glucan und/oder Lichenan bei der Reinigung von Membranen und Vorrichtungen der Lebensmittelindustrie, insbesondere der Brauereündustrie, **dadurch gekennzeichnet, daß** man ein β-glucanolytische Aktivität aufweisendes Enzym, welches die in Fig. 1 wiedergegebene Aminosäuresequenz aufweist oder eine Homologie von über 70 % zu der des Enzyms mit der in SEQ ID-NO:1 wiedergegebenen Aminosäuresequenz aufweist, einsetzt.

## Claims

1. β-Glucanase-producing microorganism *Bacillus alkalophilus* DSM 9956.

2. Enzyme with β-glucanolytic activity, **characterized in that** it has the amino acid sequence shown in Fig 1.

3. Enzyme with β-glucanolytic activity, **characterized in that** it has a homology of more than 70% to that of the enzyme with the amino acid sequence reproduced in SEQ ID-NO.:1.

4. Enzyme as claimed in claim 3, **characterized in that** it has a homology of 75% to 99% to that of the enzyme with the amino acid sequence reproduced in SEQ ID-NO:1.

5. Gene which encodes for an enzyme with β-glucanolytic activity, **characterized in that** it has the sequence reproduced in SEQ ID-NO.:2 or a homology of more than 70% to that sequence.

6. Gene as claimed in claim 5, **characterized in that** it has a homology of 75% to 99% to the sequence reproduced in SEQ ID-NO.:2.

7. Microorganism obtainable by microbiological processes which contains a gene as claimed in one of claims 5 to 6.

8. The use of an enzyme with β-glucanolytic activity which has the amino acid sequence shown in Fig. 1 for removing glucan and/or lichenan in the cleaning of membranes and equipment in the food industry, more particularly in the brewing industry.

9. The use of an enzyme with β-glucanolytic activity which has a homology of more than 70% to that of the enzyme with the amino acid sequence reproduced in SEQ ID-NO:1 for removing glucan and/or lichenan in the cleaning of membranes and equipment in the food industry, more particularly in the brewing industry.

10. The use claimed in claim 9, **characterized in that** the enzyme with β-glucanolytic activity has a homology of 75% to 99% to that of the enzyme with the amino acid sequence reproduced in SEQ ID-NO:1

11. A process for removing glucan and/or lichenan in the cleaning of membranes and equipment in the food industry, more particularly in the brewing industry, **characterized in that** an enzyme with β-glucanolytic activity which has the amino acid sequence shown in Fig. 1 or a homology of more than 70% to that of the enzyme with the amino acid sequence reproduced in SEQ ID-NO.:1 is used.

## Revendications

1. Microorganisme produisant de la β-glucanase, le Bacillus alkalophilus DSM 9956.

2. Enzyme, qui présente une activité β-glucanolytique, **caractérisée en ce qu'**elle présente la séquence d'acides aminés reproduite à la figure 1.

3. Enzyme, qui présente une activité β-glucanolytique, **caractérisée en ce qu'**elle présente une homologie de plus de 70% avec l'enzyme ayant la séquence d'acides aminés reproduite dans la séquence SEQ ID-NO:1.

4. Enzyme selon la revendication 3, **caractérisée en ce qu'**elle présente une homologie de 75% à 99% avec l'enzyme ayant la séquence d'acides aminés reproduite dans la séquence SEQ ID-NO:1.

5. Gène, qui code pour une enzyme ayant une activité β-glucanolytique, **caractérisé en ce qu'**il présente la séquence reproduite dans la séquence SEQ ID-NO:2 ou une homologie de plus de 70% avec cette séquence.

6. Gène selon la revendication 5, **caractérisé en ce qu'**il présente une homologie de 75% à 99% avec la séquence reproduite dans la séquence SEQ ID-NO:2.

7. Microorganisme pouvant être obtenu au moyen de procédés microbiologiques, lequel contient un gène selon l'une quelconque des revendications 5 à 6.

8. Utilisation d'une enzyme présentant une activité β-glucanolytique qui présente la séquence d'acides aminés reproduite à la figure 1, pour l'élimination de glucane et/ou de lichenane lors du nettoyage de membranes et de dispositifs de l'industrie alimentaire, en particulier de l'industrie brassicole.

9. Utilisation d'une enzyme présentant une activité β-glucanolytique, qui présente une homologie de plus de 70% avec l'enzyme ayant la séquence d'acides aminés reproduite dans la séquence SEQ ID-NO:1, pour l'élimination du glucane et/ou du lichenane, lors du nettoyage de membranes et de dispositifs de l'industrie alimentaire, en particulier de l'industrie brassicole.

10. Utilisation selon la revendication 9, **caractérisée en ce que**, l'enzyme présentant une activité β-glucanolytique, présente une homologie de 75% à 99% avec l'enzyme ayant la séquence d'acides aminés reproduite dans la séquence SEQ ID-NO:1.

11. Procédé pour l'élimination du glucane et/ou du lichenane lors du nettoyage de membranes et de dispositifs de l'industrie alimentaire, en particulier de l'industrie brassicole, **caractérisé en ce que** l'on met en oeuvre une enzyme présentant une activité β-glucanolytique, laquelle présente la séquence d'acides aminés reproduite à la figure 1, ou présente une homologie de plus de 70% avec l'enzyme ayant la séquence d'acides aminés reproduite dans la séquence SEQ ID-NO:1.
